# EUROPEAN PATENT APPLICATION

(11) **EP 1 389 428 A1**
(43) Date of publication of application: **18.02.2004**
(21) Application number: 03077573.8
(22) Date of filing: 14.08.2003
(51) Int. Cl.: A23K 1/16, A23L 1/30, A61K 35/78

(54) **Herb extract comprising Withania Somniferum and Astralagus Membranaceus and use thereof**

(30) Priority: 16.08.2002 NL 1021288
(71) Applicant: Exenta B.V., 5757 PG Liessel (NL)
(72) Inventor: Willems, Mario Petronella Johannes, NL-5757 PG Liessel (NL)
(74) Representative: Blokland, Arie

(57) **Abstract**

The present invention relates to a herb extract comprising Withania Somniferum and Astragalus Membranaceus. The present invention furthermore relates to the use of such a herb extract for preparing a medicine for enhancing the condition of animals, in which herb extract Withania Somniferum and Astragalus Membranaceus are present in a joint amount of at least 20 wt. %, based on the total weight of the herb extract.

## Description

The present invention relates to a herb extract comprising Withania Somniferum and Astragalus Membranaceus. The present invention furthermore relates to the use of such a herb extract for preparing a medicine for enhancing the condition of animals.

From US patent No. 6,133,323 there is known a process for enhancing immune response and improving the overall health of animals, which process comprises the administration for a specific period of time of an active amount of beta-carotene in the animal's diet. The amount of beta-carotene to be administered is about 1-50 mg/day, the other components being about 30% crude protein, about 20% fat, and about 10% total dietary fibre. Beta-carotene is known as a precursor of vitamin A, which is converted into vitamin A by enzymes in the bodies of specific mammals, such as people and canines. Felines, however, do not have this enzym and consequently they are unable to convert beta-carotene into vitamin A. Beta-carotene is further known to exhibit a potential anti-oxidant activity and protect cell membranes against oxidative damage. No mention is made in said document of the use of a special herb extract for enhancing the condition of animals, however.

From Dutch patent No. 1016519 there is known a psychotherapeutic composition containing herb extracts of Allium sativium, Juglans regia, Echinnacea purpurea and Viola tricolor, which composition is credited with a general condition and resistance enhancing effect.

Via the Internet, the firm of GetVitamins offers an "advanced immune-building system" which contains a large series of components, among which Astragalus and Withania Somniferum. The firm of Napiers Direct markets a product containing Withania, Astragalus, Echinacea, Ginkgo and Liquorice.

One aspect of the present invention is to provide a herb extract which enhances the condition of animals.

Another aspect of the present invention is to reduce the use of medicines and to substitute so-called AMGP's (antimicrobial growth promotors) in animal feeds.

Another aspect of the present invention is to provide a herb extract which stimulates the immune system of animals.

Yet another aspect of the present invention is to provide a herb extract to be used with animals, the aim being stronger growth, an increased feed conversion and a lower number of deaths, in particular among animals kept for meat production, such as fattening pigs.

Another aspect of the present invention is to provide a herb extract for animals which will lead to a higher conception rate, a larger number of young born alive per litter, and a greater weight of the sucklings upon being weaned.

Yet another aspect of the present invention is to provide a herb extract for animals which is in particular suitable for being used with laying poultry, the aim being an increase as regards the quality and also as regards the quantity of the eggs that are laid, so that the number of rejected eggs will decrease and a larger number of eggs will fall under a higher quality category.

One aspect of the present invention is furthermore to provide a herb extract for animals, which herb extract is in particular suitable for animals kept for milk production, with a the stimulation of the udder being effected, as a result of which the quantity and the quality of the milk yield will increase and the mother animal will release the milk more easily, so that the newborn animal can make better use of the beestings and there will be fewer cases of inflammation of the udder.

The invention as referred to in the introduction is characterized in that the herb extract, in addition to Withania Somniferum and Astragalus Membranaceus, furthermore comprises one or more of the following components selected from the group consisting of Allium Sativum, Trigonella Foenum, Tabebuia Impestiginosa and Taraxacum Officinale.

The invention is further characterized by a herb extract which, in addition to Withania Somniferum and Astragalus Membranaceus, furthermore comprises one or more of the following components selected from the group consisting of Allium Sativum, Trigonella Foenum, Tabebuia Impestiginosa and Taraxacum Officinale, which herb extract is intended for use as a medicine for enhancing the condition of animals. Enhancing the condition comprises strongly interwoven psychological and physical aspects, also referred to as the psychosomatic circle.

The component Withania Somniferum in particular has a regulating and supporting effect on the nervous system and the brain. In addition, the effect of Withania Somniferum can be described as tranquillising, in particular mind-stabilising.

A special function of the Astragalus Membranaceus used in the present herb extract is to stimulate the immune system, the spleen, the lungs, the liver and the urinary tract to discharge the waste materials present in the body therefrom and to help resist any attacks from microorganisms. The intended function of Astragalus Membranaceus is in particular to increase the physical resistance by improving in particular the first lines of defence of the immune system. When animal is more tranquil as a result thereof, it will be less liable to disease and it will recover more quickly from possible damage.

Preferably, Withania Somniferum and Astragalus Membranaceus are present in the present herb extract in a joint amount of at least 20 wt. %, based on the total weight of the herb extract.

If the joint amount of the aforesaid components is less than 20 wt. %, the effect will be inadequate. In order to obtain a very adequate effect of the present herb extract, Withania Somniferum and Astragalus Membranaceus are preferably present in a joint amount of at least 40 wt. %, based on the total weight of the herb extract.

Preferably, Withania Somniferum and Astragalus Membranaceus are each present in the present herb extract in an amount of at least 10 wt. %, preferably at least 20 wt. %, based on the total weight of the herb extract.

If Withania Somniferum and Astragalus Membranaceus are used in the present herb extract in an amount of less than at least 10 wt. % each, an inadequate stimulation of the immune system will result, which is undesirable in practice.

A desirable embodiment of the present herb extract furthermore comprises one or more of the following components selected from the group of Allium Sativum, Trigonella Foenum, Tabebuia Impestiginosa and Taraxacum Officinale in an amount of maximally 80 wt. %, based on the total weight of the herb extract. These herbs are credited with a favourable effect on the psychosomatic circle.

The use of Allium Sativum has a favourable effect on the blood circulation and on the digestive system, and generally an improvement as regards the digestion function and a higher intake of building materials and energy-providing materials can be achieved. In addition to that, Allium Sativum functions to prevent diarrhoea and has a favourable effect as regards the suppression of bacterial infections. Trigonella Foenum can especially be said to have a fertility-enhancing effect. Trigonella Foenum can furthermore be credited with stimulating the milk production, alleviating irritated tissue, lowering fever and improving the digestion. In addition, Trigonella Foenum functions to stimulate the healing process and to provide a laxative, expectorant and diuretic effect. Tabebuia Impestiginosa in particular has antibiotic and tumorsuppressing characteristics, and the use of this component is in particular desirable in order to strengthen the line of defence and stimulate the immune system. Furthermore, the use of this component is desirable for suppressing inflammation phenomena, cancerous growths, sores, fungal infections, rheumatic complaints, skin disease and chronically degenerative disease. Tabebuie Impestiginosa furthermore has a favourable effect with regard to reinforcing the immune system. Taraxacum Officinale can be considered to be a powerful diuretic substance, it has a high potassium salt content and in addition it has an intestine-regulating and anti-rheumatic effect. In addition to that, Taraxacum Officinale has a stimulating effect as regards the liver function, the digestion and as regards the inhibition of swellings and inflammations.

The present invention furthermore relates to the use of a herb extract as described above for preparing a medicine for enhancing the condition of animals.

The present invention will be explained in more detail hereinafter by means of a number of examples, which should not be construed as being limitative but purely as being illustrative of the present invention. Although the examples only mention cows, pigs, poultry for slaughter and horses, it should be understood that the invention is not limited to this group of animals, but that it extends to animals in general. All the data mentioned are weight data, unless stated otherwise.

### Example 1.

A herb extract was composed by preparing a 35:35:30 weight mixture of Withania Somniferum, Astragalus Membranaceus and Trigonella Foenum. The powdery herb mixture thus obtained was formed into a paste with water and added to the daily feed ration of cows. From day one the cows made a quiet, calm impression compared with a group of cows in the same cowhouse whose feed had not been supplemented with the herb extract as prepared above.

### Example 2.

In the herb extract prepared in Example 1, Trigonella Foenum was substituted for Allium Sativum in an amount of 30 wt. %, based on the total weight of the herb extract. The herb extract thus prepared was administered to pigs, which pigs did not exhibit any inflammations of the hoofs during the period that the herb extract was administered. A control group of pigs to which the aforesaid herb extract had not been administered still suffered from inflammations of the hoofs.

### Comparative Example 1.

A herb extract which only contained Alium Sativum and Withania Somniferum was administered to a group of newborn calves, the aim being to stimulate the immune system. No improvement of the immune system was observed in comparison with a group of calves to which the aforesaid herb extract had not been administered. Nor was any improvement observed in comparison with a group of calves to which the herb extract of Example 1 had been administered. Said latter group of calves exhibited a remarkably reduced susceptibility to infections after the addition of the herb extract of Example 1 to their daily feed ration.

### Example 3.

In order to examine the effect of the herb extract of Example 1 in the treatment of sows, the sows were treated with the present herb extract for at least one full cycle. The sows were administered five ml of mixture (herb extract according to Example 1: water = 2:3) once a day upon being fed. The treatment of the sows was started at the moment of weaning. The sows were treated until one week after being impregnated (± 12 days), and subsequently for a period of one week during ± the fifth week of gestation, the tenth and the eleventh week of gestation and one week before littering, respectively. The sows were also treated during the first week and the last week of the nursing period. The treatment comprised a total amount of 54 days per cycle.

The results of the control group and the treated group are shown in Table 1.

**Table 1.**

| | Sows treated with herb extract of example 1 | Untreated sows |
|---|---|---|
| Born alive/litter | 12.50 | 11.87 |
| Live weight at birth/litter | 17,977 g | 15,901 g |
| % Deaths/litter | 14.64% | 15.11% |
| Number weaned/litter | 10.74 | 10.04 |
| % Deaths after weaning | 1.80% | 2.31% |
| Growth after weaning | 373 g | 318 g |

From Table 1 it is clear that the results of the control group, viz. the sows not treated with the present herb extract, were clearly poorer for all parameters. From this it follows that the present herb extract can be used successfully, for example for increasing the number of piglets born alive per litter and for increasing the live weight at birth of piglets.

### Example 4.

In order to examine the effect of a herb extract comprising 25% Withania Somniferum, 25% Allium Sativum, 25% Tabebuia Impestiginosa en 25% Astragalus Membranaceus in the treatment of fattening pigs, the Applicant treated 2 groups of fattening pigs with the present herb extract, whilst 2 groups of fattening pigs were not treated therewith. For the rest, all the animals were tended in exactly the same manner and received the same types of feed. An amount of 150 ml of herb extract was mixed with 9850 ml of water, and subsequently the mixture was connected to a medication dose-measuring device set at 1%, with an amount of 0.15 ml of herb extract per litre of drinking water being administered to the fattening pigs. Said fattening pigs were treated in weeks 1 and 2, weeks 5 and 6 and in week 9 of the fattening period.

The differences between the control group and the treated group are summarised in Table 2.

**Table 2.**

| | Untreated groups | Groups treated with the herb extract of Example 4 |
|---|---|---|
| Number of piglets | 200 | 208 |
| Average weight | 29.13 kg | 33.90 kg |
| Feed consumption animal/day | 1.96 kg | 2.03 kg |
| EW intake animal/day | 2.16 | 2.23 |
| Growth | 748 g | 777 g |
| Feed conversion 23-83 kg | 2.49 | 2.40 |
| EW conversion 23-83 kg | 2.76 | 2.67 |
| % Deaths | 8.0% | 2.9% |
| % Meat | 56.9% | 56.4% |
| % Class AA | 17.7% | 17.1% |
| % Class A | 77% | 76.6% |
| % Class B | 5.4% | 6.0% |

From Table 2 it follows that the growth, amongst other things, of the fattening pigs treated with the herb extract of Example 4 is significantly higher than that of the control group not treated with said herb extract. In addition, the percentage of deaths of the treated group of fattening pigs is significantly lower than that of the untreated group of fattening pigs. From this it follows that the present herb extract has a positive and clearly measurable effect in the treatment of fattening pigs.

### Example 5.

A herb extract comprising 25 wt.% Allium Sativum, 15 wt.% Astragalus Membranaceus, 15 wt.% Withania Somniferum, 25 wt.% Taraxacum Officinale and 20 wt.% Tabebuia Impestiginosa was added to the drinking water system of table poultry for a period of a few weeks. After a few days already, a decrease of the number of deaths and of the incidence of cannibalism could be observed in comparison with a comparable group of table poultry to which the herb extract had not been administered. The reduction of the number of deaths in comparison with the control group of table poultry amounted to 25%.

### Example 6.

A jumper that did not look too healthy was daily administered a herb extract comprising 35 wt.% Withania Somniferum, 35 wt.% Astragalus Membranaceus and 30 wt.% Taraxacum Officinale for a period of 50 days. After a period of about two weeks, the horse started to look healthier and healthier, its fur became more shiny and it ate up all of its daily feed rations. After a period of four weeks, the jumper could be cautiously ridden again and about two weeks later the animal already jumped the first obstacles. Taraxacum Officinale is credited with stimulating the body's waste-processing organs, as a result of which such waste materials can be discharged from the body more easily.

### Example 7.

The herb extract of Example 2 was administered to a newly put-out brace of broilers, mainly to prevent the incidence of diarrhoea among said animals. Usually, an increased number of deaths caused by diarrhoea is observed during the period around 10-12 days after the broilers have been put out. The number of deaths among the boilers treated with the present herb extract remained at a remarkably low level. After all, the incidence of diarrhoea is low and the net result is considerably higher than that of rearing farms that do not administer the present herb extract to their broilers. The present Applicant attributes this favourable result, without being bound by such an explanation, to a favourable effect on the psychosomatic circle, in particular by enhancing the digestion function (more building materials and energy-providing materials), as a result of which the animals require less food for combusting their fats and get energy in return.

### Example 8.

A herb extract comprising 30% Withania Somniferum, 30% Astragalus Membranaceus and 40% Tabebuia Impestiginosa was administered to a seriously ill pair of animals via the drinking water. An additional examination had shown that said animals had contracted a very serious bacterial infection, and it was first attempted to increase the physical resistance of the animals, in particular by improving the first lines of defence of the immune system, primarily with Astragalus Membranaceus. In addition to that, their mental resistance against stress and their physical stamina were increased with Withania Somniferum. The favourable psychosomatic circle, which is effected by the addition of said two herbs, in combination with a strong inhibition of the bacterial growth obtained by the presence of Tabebuia Impestiginosa, eventually caused the bacterial climate among said animals to return to normal. If the immune system shows any sign of response, a stimulation thereof will be effected by using the present herb extract, and the animals will recover. If the condition of the animals has worsened very badly, so that the immune system does not show any sign of response anymore, the favourable effect of the present herb extract will not become evident.

### Example 9.

A herb extract comprising 35% Withania Somniferum, 20% Astragalus Membranaceus, 25% Taraxacum Officinale and 20% Tabebuia Impestiginosa was administered to pigs in a pig house, and the results were compared with those of a group of pigs that had not been administered the present herb extract. The measuring data showed that the pigs of the group that had been administered the present herb extract made a very tranquil impression, they quietly lay down on the ground much sooner, clearly feeling more at ease, whilst the incidence of ear and tail biting was minimized. In the control group it was evident that the animals did not feel very well, the animals look stressed and exhibited a tendency towards cannibalism.

### Example 10.

A herb extract comprising 40% Withania Somniferum, 20% Astragalus Membranaceus and 40% Trigonella Foenum was administered to young boars in a rearing house, which young boars had a body weight which varied from 25 kg to 130 kg. The administration of the aforesaid herb extract led to an improved development of the testicles in comparison with a control group, to which the present herb extract was not administered, whilst in addition an improvement both with regard to the quality and with regard to the quantity of the sperm obtained from said animals was observed. The processing frequency of the examined sperm cells appeared to be much higher than that of the untreated boars from the control group. In addition, it has become apparent that the boars used at so-called A.I. centres generally appeared to be in a much better condition for successfully mounting the dummy sow, whilst furthermore they produced sperm of a better quality.

### Example 11.

The same herb extract as prepared in Example 10 was administered to young sows having a body weight varying from 25 kg to 120 kg. This group of sows was compared with a control group comprising the same number of sows. At an age of about 8 months the sows, which by then had a body weight of about 130 kilos, were impregnated with the same sperm as the sows in the control group. The conception rate of the sows that had received the present herb extract was 91%, compared to a conception rate of 86% of the sows from the control group. From these data it appears that the present herb extract has an advantageous effect on the conception rate. Measuring data furthermore show that the sows treated with the present herb extract on average dropped 12.3 live piglets in comparison with an average number of 11.9 piglets born alive from the sows in the control group. When these data are converted to an average sow farm counting 500 sows, this means that about 500 more piglets will be born on an annual basis, which is advantageous. The same herb extract has moreover been used as a veterinary treatment of inflammations of, for example, the udder and the uterus, and it has been found that the recovery rate of the control group was clearly lower than that of the sows treated with the present herb extract.

### Example 12.

A herb extract with a base of Astralagus Membranaceus in combination with Withania Somniferum and Tabebuia Impestiginosa, each in the same weight percentage, was used for healing inflamed wounds both on the legs and on the udder of a dairy cow that had injured itself on barbed wire. Said wounds had become inflamed after a few days and had started to fester. After the wounds had been cleaned with previously boiled water, they were treated with the aforesaid herb extract in the form of an ointment twice a day for a period of two weeks. After a few days of treatment the wound looked much cleaner already, and the formation of pus had stopped after a week already. In the second week after treatment the wounds had nicely healed, and the cow could soon be milked together with the other cows again. The present herb extract thus has a healing effect, which is mainly attributed to the self-protecting properties that curb the effect of the damage to the cell membrane.

## Claims

1. A herb extract which, in addition to Withania Somniferum and Astragalus Membranaceus, furthermore comprises one or more of the following components selected from the group consisting of Allium Sativum, Trigonella Foenum, Tabebuia Impestiginosa and Taraxacum Officinale.

2. A herb extract which, in addition to Withania Somniferum and Astragalus Mebranaceus, furthermore comprises one or more of the following components selected from the group consisting of Allium Sativum, Trigonella Foenum, Tabebuia Impestiginosa and Taraxacum Officinale, which herb extract is intended for use as a medicine for enhancing the condition of animals.

3. A herb extract according to any one or more of the preceding claims, **characterized in that** Withania Somniferum and Astragalus Membranaceus are present in a joint amount of at least 20 wt. %, based on the total weight of the herb extract.

4. A herb extract according to any one or more of the preceding claims, **characterized in that** Withania Somniferum and Astragalus Membranaceus are present in a joint amount of at least 40 wt. %, based on the total weight of the herb extract.

5. A herb extract according to any one or more of the preceding claims, **characterized in that** Withania Somniferum and Astragalus Membranaceus are each present in an amount of at least 10 wt. %, based on the total weight of the herb extract.

6. A herb extract according to any one or more of the preceding claims, **characterized in that** Withania Somniferum and Astragalus Membranaceus are each present in an amount of at least 20 wt. %, based on the total weight of the herb extract.

7. A herb extract according to any one or more of the preceding claims, **characterized in that** one or more of the following components selected from the group of Allium Sativum, Trigonella Foenum, Tabebuia Impestiginosa and Taraxacum Officinale are present in an amount of maximally 80 wt. %, based on the total weight of the herb extract.

8. Use of a herb extract as defined in any one or more of the preceding claims for preparing a medicine for enhancing the condition of animals.
